Europäisches Patentamt

European Patent Office

Office européen des brevets

(11) Veröffentlichungsnummer: **0 477 420 A1**

# (12) EUROPÄISCHE PATENTANMELDUNG

(21) Anmeldenummer: **90118714.6**

(22) Anmeldetag: **28.09.90**

(51) Int. Cl.5: **A61B 5/00**, A61N 1/36

(43) Veröffentlichungstag der Anmeldung:
**01.04.92 Patentblatt 92/14**

(84) Benannte Vertragsstaaten:
**DE FR GB IT NL SE**

(71) Anmelder: **Siemens Elema AB**
**Röntgenvägen 2**
**S-171 95 Solna 1(SE)**
(84) **SE**

(71) Anmelder: **SIEMENS AKTIENGESELLSCHAFT**
**Wittelsbacherplatz 2**
**W-8000 München 2(DE)**
(84) **DE FR GB IT NL**

(72) Erfinder: **Lekholm, Anders, Dr.-Ing.**
**Gnejsvägen 4**
**S-161 39 Bromma(SE)**

(54) **Messvorrichtung zur intrakardialen Erfassung eines der körperlichen Aktivität eines Lebewesens entsprechenden Messsignales.**

(57) Eine Meßvorrichtung zur intrakardialen Erfassung eines der körperlichen Aktivität eines Lebewesens entsprechenden Meßsignales enthält eine optoelektronische Einrichtung (16, 17) zur Messung des Blutsauerstoffgehaltes und einen Temperatursensor (18) zur Messung der Bluttemperatur. Die Meßvorrichtung erzeugt wahlweise ein blutsauerstoffgehalt- und bluttemperaturabhängiges Meßsignal, ein nur blutsauerstoffgehaltabhängiges Meßsignal oder ein nur bluttemperaturabhängiges Meßsignal.

FIG 2

Die Erfindung betrifft eine Meßvorrichtung zur intrakardialen Erfassung eines der körperlichen Aktivität eines Lebewesens entsprechenden Meßsignales.

Derartige Meßvorrichtungen werden beispielsweise in der Herzschrittmacher-Technologie benötigt, um die Stimulationsfrequenz, mit der das Herz des den Herzschrittmacher tragenden Patienten im Bedarfsfalle stimuliert wird, der körperlichen Aktivität des Patienten anpassen zu können. Eine Vorrichtung dieser Art ist beispielsweise in der EP-A-0 249 822 beschrieben. Die bekannte Meßvorrichtung enthält eine Einrichtung zur Bildung eines dem Blutsauerstoffgehalt entsprechenden Signalanteiles mit einem Lichtsender zur Abstrahlung von Licht in das Blut des Lebewesens und einem Lichtempfänger zum Empfangen des am Blut reflektierten Lichtes, einen Temperatursensor zur Bildes eines der Bluttemperatur entsprechenden Signalanteiles und eine Versorgungsschaltung. Da der Blutsauerstoffgehalt bei einer gewissen Intensität der körperlichen Aktivität in einen Sättigungszustand übergeht, kann der dem Blutsauerstoffgehalt entsprechende Signalanteil die körperliche Aktivität nur bei relativ geringer Intensität hinreichend genau beschreiben. Andererseits stellt bei körperlicher Aktivität erhöhter Intensität die Körper- bzw. Bluttemperatur einen recht genauen Indikator für die Intensität der körperlichen Aktivität dar. Im Falle der bekannten Meßvorrichtung werden daher der dem Blutsauerstoffgehalt entsprechende Signalanteil und der der Bluttemperatur entsprechende Signalanteil in einer solchen Weise überlagert, daß sich auch bei körperlicher Aktivität höherer Intensität ein gut auswertbares Meßsignal ergibt. Damit besitzt die bekannte Meßvorrichtung zwar eine Charakteristik, die denen von ebenfalls bekannten Meßvorrichtungen, die entweder nur dem Blutsauerstoffgehalt oder nur der Bluttemperatur entsprechende Signale liefern, unter bestimmten Bedingungen vorzuziehen ist, aber auch die Charakteristika der nur blutsauerstoffgehalt- bzw. bluttemperaturabhängig arbeitenden Meßvorrichtungen haben ihre Vorzüge. Bei der bekannten Meßvorrichtung besteht übrigens die Möglichkeit, entweder nur den Lichtsender, den Lichtempfänger und den Temperatursensor in einen in das Herz des Patienten einzuführenden Katheter bzw. die Elektrode eines Herzschrittmachers zu integrieren oder außerdem auch die Ansteuerschaltung in den Katheter bzw. die Elektrode zu integrieren. Im ersten Fall ergeben sich zwar keine Bauraumprobleme, da es sich sowohl bei dem Lichtsender als auch bei dem Lichtempfänger und dem Temperatursensor um sehr kleine Bauteile handelt, jedoch muß der Katheter eine wenigstens vierpolige Leitung enthalten bzw. eine Elektrode mit wenigstens vier Leitern verwendet werden. Im zweiten Falle genügt es zwar, wenn der Katheter eine zweipolige Leitung enthält bzw. eine Elektrode mit zwei Leitern verwendet wird, jedoch ergeben sich Bauraumprobleme, da zusätzlich die Ansteuerschaltung in den Katheter bzw. die Elektrode integriert sein muß.

Der Erfindung liegt die Aufgabe zugrunde, eine Meßvorrichtung der eingangs genannten Art so auszubilden, daß deren Charakteristik den jeweiligen Bedürfnissen und der Intensität der jeweils vorliegenden körperlichen Aktivität möglichst vielseitig anpaßbar ist. Der Erfindung liegt außerdem die Aufgabe zugrunde, eine Meßvorrichtung der eingangs genannten Art so auszubilden, daß zwischen Lichtsender, Lichtempfänger und Temperatursensor einerseits und Ansteuerschaltung andererseits eine möglichst geringe Anzahl von elektrischen Verbindungen benötigt wird.

Nach der Erfindung wird diese Aufgabe durch eine Meßvorrichtung zur intrakardialen Erfassung eines der körperlichen Aktivität eines Lebewesens entsprechenden Meßsignales gelöst, welche eine Einrichtung zur Bildung eines dem Blutsauerstoffgehalt entsprechenden Signalanteiles mit einem Lichtsender zur Abstrahlung von Licht in das Blut des Lebewesens und einem Lichtempfänger zum Empfangen des am Blut reflektierten Lichtes, einen Temperatursensor zur Bildung eines der Bluttemperatur entsprechenden Signalanteiles und eine Ansteuerschaltung aufweist, welche einen konstanten Meßstrom oder eine konstante Meßspannung abgibt, wobei der Lichtsender, der Lichtempfänger und der Temperatursensor zu einem Zweipol verbunden sind, durch den der Meßstrom fließt oder über dem die Meßspannung abfällt, und wobei sich das Meßsignal aus dem Spannungsabfall über der Ansteuerschaltung oder aus dem der Ansteuerschaltung entnommenen Strom ergibt. Da sich in Abhängigkeit von dem Strom bzw. der Spannung, die die Ansteuerschaltung abgibt, zum einen die Intensität des von dem Lichtsender ausgehenden Lichtes ändert und zum anderen infolge der temperaturabhängigen Bauteilcharakteristika der Elemente der Einrichtung zur Bildung eines dem Blutsauerstoffgehalt entsprechenden Signalanteiles der durch diese Einrichtung fließende Strom bzw. die über dieser Einrichtung abfallende Spannung temperaturabhängig ist, lassen sich unterschiedliche Charakteristika der Meßvorrichtung realisieren. Dabei wird in Abhängigkeit von dem jeweils gewählten Meßstrom bzw. der jeweils gewählten Meßspannung entweder der Einfluß des dem Blutsauerstoffgehalt entsprechenden oder der des der Bluttemperatur entsprechenden Signalanteiles überwiegen oder aber eine beide Anteile des Meßsignales gleichermaßen berücksichtigende Charakteristik vorliegen. Infolge des Umstandes, daß der Lichtsender, der Lichtempfänger und der Temperatursensor zu einem Zweipol zusammengeschaltet sind, genügen zwischen diesem Zweipol und der Ansteuerschaltung zwei Verbindungsleitungen. Dies ist deshalb vorteilhaft, weil eine zweipolige Leitung leicht in einen Katheter integriert werden kann bzw. die Verwendung von Elektroden mit zwei Leitern problemlos ist. Es besteht im Falle der erfindungsgemäßen Meßvorrichtung somit keine Notwendigkeit, die Ansteuerschaltung in den Katheter bzw. die Elektrode zu integrieren, so daß hiermit verbundene Bauraumprobleme vermieden sind.

Um die Charakteristik der Meßvorrichtung während des Betriebes sich verändernden Bedürfnissen anpassen zu können, sieht eine Variante der Erfindung vor, daß Mittel zum Verstellen des Meßstromes oder der Meßspannung vorgesehen sind. Eine besonders bevorzugte Variante der Erfindung sieht vor, daß die Einrichtung zur Bildung des dem Blutsauerstoffgehalt entsprechenden Signalanteiles ein Bauteil mit einem dem des Temperatursensors entgegengerichteten Temperaturkoeffizienten aufweist und der Meßstrom oder die Meßspannung auf einen Wert einstellbar ist, bei dem das Meßsignal bluttemperaturunabhängig ist. Eine weitere bevorzugte Variante der Erfindung sieht vor, daß der Meßstrom oder die Meßspannung auf einen Wert einstellbar ist, bei dem die optische Funktion der Einrichtung zur Bildung des dem Blutsauerstoffgehalt entsprechenden Signalanteiles so weit reduziert ist, daß das Meßsignal blutsauerstoffgehaltunabhängig ist. Es besteht also in vorteilhafter Weise auch die Möglichkeit, die Meßvorrichtung in einer solchen Weise zu betreiben, daß ihre Charakteristik der einer ausschließlich blutsauerstoffgehalt- oder bluttemperaturabhängig arbeitenden Meßvorrichtung entspricht.

Ausführungsformen der Erfindung sehen vor, daß als Lichtsender und/oder Lichtempfänger Fotohalbleiter und als Temperatursensor ein Bauelement mit einem temperaturabhängigen Widerstandswert vorgesehen sind. Derartige Bauteile sind sehr gut miniaturisierbar und von daher leicht in einen in das Herz des jeweils zu untersuchenden Lebewesens einführbaren Katheter oder eine intrakardiale Elektrode integrierbar. Außerdem besitzen diese Bauelemente eine lange Lebensdauer und während ihrer Lebensdauer weitgehend konstante Bauteilcharakteristika.

Eine besonders bevorzugte Variante der Erfindung sieht vor, daß als Lichtsender eine Leuchtdiode und als Temperatursensor ein zu dieser in Serie geschalteter PTC-Widerstand vorgesehen ist, daß als Lichtempfänger ein Fototransistor vorgesehen ist, der dem PTC-Widerstand parallel geschaltet ist, und daß die Ansteuerschaltung einen konstanten Meßstrom liefert. Da die Durchlaßspannung der Leuchtdiode im wesentlichen stromunabhängig ist, jedoch eine Temperaturabhängigkeit mit einem negativen Temperaturkoeffizienten aufweist, läßt sich sehr leicht ein Wert des Meßstromes finden, bei dem sich die entgegengesetzten Temperaturgänge der Leuchtdiode und des PTC-Widerstandes aufheben und die Meßvorrichtung somit ein temperaturabhängiges Meßsignal liefert. Außerdem kann der Meßstrom leicht auf einen so niedrigen Wert abgesenkt werden, daß die Leuchtdiode kein Licht oder Licht so geringer Intensität aussendet, daß das Meßsignal blutsauerstoffgehaltunabhängig ist.

Eine Ausführungsform der Erfindung sieht vor, daß die Ansteuerschaltung den Meßstrom oder die Meßspannung pulsförmig abgibt. Hierdurch wird erreicht, daß infolge des Stromdurchganges durch die Meßvorrichtung keine das Meßsignal verfälschende Erwärmung der Meßvorrichtung auftreten kann.

Die erfindungsgemäße Meßvorrichtung wird vorzugsweise in einem Herzschrittmacher verwendet, dessen Stimulationsfrequenz in Abhängigkeit von dem Meßsignal gesteuert wird.

Die Erfindung wird nachfolgend anhand der beigefügten Zeichnungen erläutert. Es zeigen:

Fig. 1 in schematischer Darstellung einen eine erfindungsgemäße Meßvorrichtung enthaltenden Herzschrittmacher, und

Fig. 2 und 3 Prinzipschaltbilder erfindungsgemäßer Meßvorrichtungen.

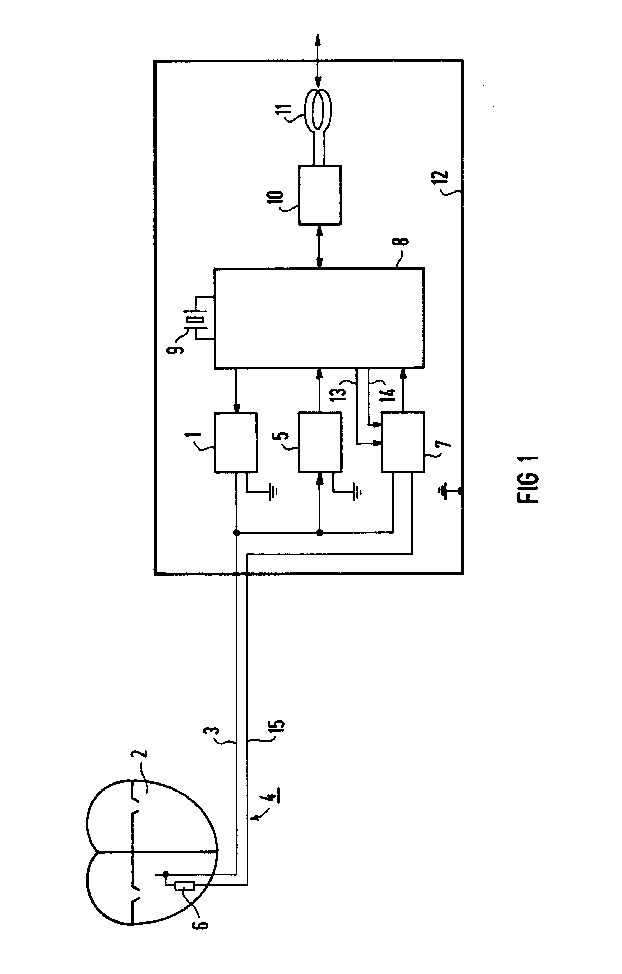

Der Herzschrittmacher gemäß Fig. 1 enthält in an sich bekannter Weise einen Stimulationsimpuls-Generator 1 zur Erzeugung von elektrischen Stimulationsimpulsen. Diese sind dem schematisch angedeuteten zu stimulierenden Herz 2 über den einen Leiter 3 einer zwei Leiter aufweisenden intrakardiellen Elektrode zugeführt, die insgesamt mit 4 bezeichnet ist. Diese ist mit ihrem freien Ende in das durch das Venensystem zugängliche rechte Ventrikel des Herzens 2 eingepflanzt.

Außerdem ist in ebenfalls an sich bekannter Weise eine Detektoreinrichtung 5 zur Detektion natürlicher Herzschläge vorgesehen, deren Eingang ebenfalls mit dem Leiter 3 der Elektrode 4 verbunden ist.

Weiter ist in an sich bekannter Weise eine Vorrichtung zur Bildung eines der körperlichen Aktivität des den Herzschrittmacher tragenden Patienten entsprechenden Meßsignales dienende Meßvorrichtung vorgesehen, die einen an dem in das Herz 2 eingepflanzten Ende der Elektrode 3 angeordneten Sensor 6 und eine Ansteuerschaltung 7 für den Sensor 6 aufweist.

Der Stimulationsimpulsgenerator 1, die Detektoreinrichtung 5 und die Ansteuerschaltung 7 sind mit einer Steuerlogik 8 verbunden, an die außerdem zur Erzeugung der zum Betrieb des Herzschrittmachers erforderlichen Takt- und Zeitsignale ein Quarz 9 angeschlossen ist. An die Steuerlogik 8 ist außerdem in an sich bekannter Weise ein Telemetrieschaltkreis 10 angeschlossen, der mit einer Sende/Empfangs-Spule 11 verbunden ist. Der Herzschrittmacher, dessen elektrische und elektronische Bauelemente in einem implantierbaren, hermetisch dichten Gehäuse 12 aufgenommen sind, kann also mit einem in Fig. 1 nicht dargestellten externen Gerät, einem sogenannten Programmer, zum Austausch und zur Veränderung von in der Steuerlogik 8 des Herzschrittmachers gespeicherten Daten kommunizieren, indem die Sende/Empfangs-Spule des Programmers relativ zu der des Herzschrittmachers derart angeordnet wird, daß beide induktiv gekoppelt sind.

Da der Stimulationsimpuls-Generator 1 und die Detektoreinrichtung 5 mit dem zu stimulierenden Herz 2 nur über den einen Leiter 3 der Elektrode 4 in Verbindung stehen, wird das Bezugspotential über das elektrisch leitende Gehäuse 12 des Herzschrittmachers an den Körper des Patienten angelegt. Dies ist dadurch verdeutlicht, da sowohl dem Gehäuse 12 als auch jeweils einem Anschluß des Stimulationsimpuls-Generators 1 und der Detektoreinrichtung 5 Massesymbole zugeordnet sind.

Die Steuerlogik 8 steuert das Zusammenwirken des Stimulationsimpuls-Generators 1, der Detektoreinrichtung 5 und des Sensors 6 samt Ansteuerschaltung 7 derart, daß der Stimulationsimpuls-Generator 1 einen Stimulationsimpuls dann abgibt, wenn vor Ablauf eines bestimmten Zeitintervalles, dessen Dauer von der mittels des Sensors 6 und der Ansteuerschaltung 7 ermittelten körperlichen Aktivität des Patienten abhängt, mittels der Detektoreinrichtung 5 kein natürlicher Herzschlag detektiert wurde. Dabei verändert die Steuerlogik 8 die Dauer des definierten Zeitintervalles derart, daß diese mit zunehmender körperlicher Aktivität geringer wird. Die Stimulationsfrequenz, mit der im Bedarfsfalle aufeinanderfolgende Stimulationsimpulse abgegeben werden, steigt also mit zunehmender körperlicher Aktivität. So ist sichergestellt, daß die Herzschlagfrequenz des stimulierten Herzens 2 ähnlich wie bei einem gesunden Herz unter zunehmender körperlichen Belastung ansteigt und bei abnehmender körperlichen Belastung wieder zurückgeht. Nähere Einzelheiten betreffend Herzschrittmacher, deren Stimulationsfrequenz belastungsabhängig gesteuert wird, können der Druckschrift "Sensorcontrolled Pulse Generator SENSOLOG 703" - Physician's Manual, Siemens-Elema AB, Pacemaker Division, Solna, Sweden, entnommen werden.

Bei dem Sensor 6 handelt es sich um einen Sensor, welcher im Zusammenwirken mit der Ansteuerschaltung 7, mit der er außer mit dem Leiter 3 auch mit einem zweiten Leiter 15 der Elektrode 4 verbunden ist, ein der Steuerlogik 8 von der Ansteuerschaltung 7 zugeführtes Meßsignal erzeugt, das einen dem Blutsauerstoffgehalt entsprechenden Signalanteil und/oder einen der Bluttemperatur entsprechenden Signalanteil enthalten kann. Über eine von der Steuerlogik 8 zu der Ansteuerschaltung 7 führende Steuerleitung 13 kann die Steuerlogik 8 die Ansteuerschaltung 7 derart beeinflussen, daß diese mit dem Sensor wahlweise derart zusammenwirkt, daß ein von Blutsauerstoffgehalt und Bluttemperatur abhängiges Meßsignal, ein nur vom Blutsauerstoffgehalt abhängiges Meßsignal oder ein nur von der Bluttemperatur abhängiges Meßsignal zur Verfügung steht. Es sind also drei grundsätzlich unterschiedliche Charakteristiken einstellbar, nach denen das die körperliche Aktivität des den Herzschrittmacher tragenden Patienten wiedergebende Meßsignal gebildet werden kann. Die Umschaltung der Charakteristik kann mittels des Programmers auf telemetrischem Wege vorgenommen werden. Außerdem ist die Steuerlogik 8 derart ausgebildet, daß sie in der Lage ist, in Abhängigkeit von der Intensität der jeweils vorliegenden körperlichen Aktivität die geeignete Charakteristik einzustellen. In dieser Betriebsart, die ebenfalls auf telemetrischem Wege einstellbar ist, überwacht die Steuerlogik 8 die Herzschlag- bzw. Stimulationsfrequenz und stellt für niedrige körperliche Aktivitäten die ausschließlich vom Blutsauerstoffgehalt abhängige Charakteristik und für sehr hohe körperliche Aktivitäten die ausschließlich temperaturabhängige Charakteristik ein. In einem Übergangsbereich mittlerer körperlicher Aktivität stellt die Steuerlogik 8 eine sowohl blutsauerstoffgehalt- als auch bluttemperaturabhängige Charakteristik ein.

Über die Steuerleitung 14 setzt die Steuerlogik 8 die Ansteuerschaltung 7 im Anschluß an die Detektion eines natürlichen Herzschlages bzw. die Abgabe eines Stimulationsimpulses jeweils für diejenige Zeit in Betrieb, die erforderlich ist, und das Meßsignal zu bilden. Auf diese Weise ergibt sich ein stromsparender Betrieb der Meßvorrichtung, da diese nur intermittierend betrieben wird. Während desjenigen Zeitraumes, in dem die Meßvorrichtung aktiv ist, sind übrigens der Stimulationsimpuls-Generator 1 und die Detektoreinrichtung 5 von dem Leiter 3 in nicht dargestellter Weise getrennt. Die Meßvorrichtung ist vorzugsweise während der absoluten Refraktärzeit (siehe hierzu das obengenannte Physician's Manual) aktiv, in der ohnehin weder Stimulationen noch Detektionen erfolgen.

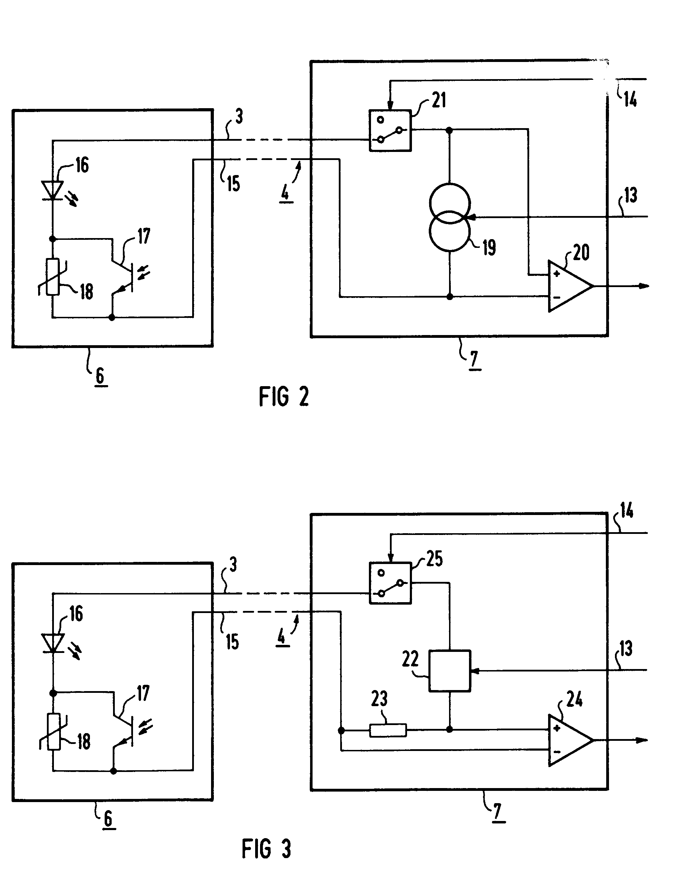

In Fig. 2 ist die erfindungsgemäße Meßvorrichtung mit dem mittels der Elektrode 4 mit der Ansteuerschaltung 7 verbundenen Sensor 6 dargestellt. Dabei sind aus Gründen der Übersichtlichkeit weder die Verbindungen des Leiters 3 mit dem Stimulationsimpuls-Generator 1 und der Detektoreinrichtung 5 noch das Ende des Leiters 3, das bei implantierter Elektrode 4 mit dem zu stimulierenden Herzmuskelgewebe in Eingriff steht, dargestellt. Der Sensor 6 enthält eine Einrichtung zur Bildung eines den Blutsauerstoffgehalt des den Herzschrittmacher tragenden Lebewesens, welcher als Lichtsender eine rote LED 16 und einen npn-Fototransistor 17 als Lichtempfänger aufweist. Die Leuchtdiode 16 und der Fototransistor 17 sind in nicht gezeigter Weise derart in die Elektrode integriert, daß die Leuchtdiode 16 ihr Licht in das venöse Blut des Patienten abstrahlt und der Fototransistor 17 den am venösen Blut reflektierten Anteil des abgestrahlten Lichtes empfängt. Die Intensität des reflektierten Lichtes stellt ein Maß für den Blutsauerstoffgehalt des venösen Blutes dar. Wegen näherer Einzelheiten wird auf die DE-PS 3 152 963 verwiesen.

Außerdem enthält der Sensor 6 einen PTC-Widerstand 18, der dazu dient, einen der Bluttemperatur des venösen Blutes entsprechenden Signalanteil zu bilden, und in nicht gezeigter Weise derart in die Elektrode integriert ist, daß eine gute Wärmeleitung zwischen dem Blut und dem PTC-Widerstand gegeben ist.

Die genannten Bauelemente des Sensors 6 sind in der Weise zu einem Zweipol verbunden, daß die Leuchtdiode 16 und der PTC-Widerstand 18 in Serie geschaltet sind und der Fototransistor 17 dem PTC-Widerstand 18 parallel geschaltet ist.

Der beschriebene Zweipol ist über die Leiter 3 und 15 der Elektrode 4 mit der Ansteuerschaltung 7 verbunden, die eine mit den genannten Leitungen verbundene Konstantstromquelle 19 enthält. Der von der Konstantstromquelle 19 gelieferte Meßstrom ist, so wie dies in Fig. 2 schematisch angedeutet ist, über die Steuerleitung 13 einstellbar. Die über dem Sensor 6 infolge des von der Konstantstromquelle 19 gelieferten Meßstromes abfallende Spannung wird mittels eines in Fig. 2 schematisch angedeuteten Operationsverstärkers 20, dessen Eingänge mit den Leitern 3 und 15 verbunden sind, verstärkt und als Meßsignal der in Fig. 2 nicht dargestellten Steuerlogik 8 zugeführt. Um den Stromfluß durch den Sensor 6 auf diejenigen Zeitintervalle beschränken zu können, in denen die Meßvorrichtung aktiv ist, enthält die Ansteuerschaltung 7 einen elektronischen Schalter 21, mittels dessen der Leiter 3 von der Konstantstromquelle 19 getrennt werden kann. Der elektronische Umschalter 21 wird von der Steuerlogik 8 über die Steuerleitung 14 in der bereits beschriebenen Weise betätigt.

Wird der von der Konstantstromquelle 19 gelieferte Meßstrom, der im folgenden mit i bezeichnet ist, auf Werte von höchstens 0,2 mA begrenzt, strahlt die Leuchtdiode 16 nicht mehr genügend Licht ab, als daß noch ein dem Blutsauerstoffgehalt entsprechender Signalanteil gebildet werden könnte. Demzufolge wird also dann, wenn der Sensor 6 mit einem Meßstrom i von höchstens 0,2 mA betrieben wird, nur der bluttemperaturabhängige Signalanteil gebildet mit der Folge, daß eine ausschließlich bluttemperaturabhängige Charakteristik der Meßeinrichtung vorliegt.

Da die Leuchtdiode 16 eine Durchlaßspannung $V_{LED}$ aufweist, die im wesentlichen von dem durch die Leuchtdiode 16 fließenden Meßstrom unabhängig ist, jedoch einen Temperaturkoeffizienten $TC_{LED}$ von $-2,5 \times 10^{-3}$ V/°C aufweist, der demzufolge vorzeichenmäßig dem Temperaturkoeffizienten $TC_{PTC}$ von beispielsweise $0,8 \times 10^{-2}$ 1/°C entgegengesetzt ist, läßt sich ein Wert für den Meßstrom i finden, bei dem sich die Temperaturgänge der Leuchtdiode 16 und des PTC-Widerstandes 18 gerade aufheben. Dies ist dann der Fall, wenn gilt

$$\Delta V_{PTC} + \Delta V_{LED} = 0 \qquad (1),$$

wobei $\Delta V_{PTC}$ und $\Delta V_{LED}$ die durch eine definierte Temperaturänderung $\Delta T$ jeweils bewirkten Änderungen des Spannungsabfalls über dem PTC-Widerstand 18 und der Leuchtdiode 16 sind. Es gilt weiter

$$\Delta V_{PTC} = i \cdot (\Delta T \cdot TC_{PTC} \cdot R_{PTC}) \qquad (2)$$

und

$$\Delta V_{LED} = \Delta T \cdot TC_{LED} \qquad (3),$$

wobei $R_{PTC}$ den Nennwiderstand des PTC-Widerstandes 18, beispielsweise $10^3$ Ohm, darstellt.

Durch Einsetzen von (2) und (3) in (1) erhält man

$$i \cdot (\Delta T \cdot TC_{PTC} \cdot R_{PTC}) + \Delta T \cdot TC_{LED} = 0 \qquad (4)$$

Nach Kürzen mit $\Delta T$, Auflösen nach i und Einsetzen der genannten Zahlenwerte ergibt sich

$$i = (2,5 \times 10^{-3})/(0,8 \times 10^{-2} \times 10^3) = 0,3125 \text{ mA} \qquad (5)$$

Es wird also deutlich, daß die beschriebene Meßeinrichtung eine praktisch temperaturunabhängige, d.h. ausschließlich blutsauerstoffgehaltabhängige Charakteristik aufweist, wenn die Steuerlogik 8 die Konstantstromquelle 19 auf einen Meßstrom i von etwa 0,3 mA einstellt.

Es versteht sich, daß die Meßeinrichtung bei mittels der Steuerlogik 8 eingestellten Meßströmen i, die größer als 0,2 mA sind und von 0,3 mA abweichen, ein sowohl blutsauerstoffgehaltals auch bluttemperaturabhängiges Meßsignal liefert.

Die in Fig. 3 dargestellte Meßanordnung unterscheidet sich von der zuvor beschriebenen dadurch, daß die Ansteuerschaltung 7 eine Konstantspannungsquelle 22 enthält, mittels derer über die Leitungen 3 und 15 der Elektrode 4 dem Sensor 6 eine konstante Meßspannung zugeführt wird, die über die Steuerleitung 13 einstellbar ist. In die Leitung 15 ist ein Shunt-Widerstand 23 geschaltet. Die über dem Shunt-Widerstand 23 abfallende Spannung, die dem durch den Sensor 6 fließenden Strom entspricht, wird mittels eines schematisch angedeuteten Operationsverstärkers 24 verstärkt und der Steuerlogik 8 zugeführt. Außerdem enthält die Ansteuerschaltung 7 einen elektronischen Schalter 25, der zwischen der Konstantspannungsquelle 22 und dem Leiter 3 liegt und über die Steuerleitung 14 von der Steuerlogik 8 derart gesteuert wird, daß eine leitende Verbindung der Konstantspannungsquelle 22 mit dem Leiter 3 nur dann vorliegt, wenn das Meßsignal tatsächlich gebildet werden soll. Auf diese Weise erfolgt ein Stromdurchgang durch den Sensor 6 nur dann, wenn tatsächlich das Meßsignal gebildet werden soll.

Infolge des Umstandes, daß die Bauteile des Sensors 6 sowohl im Falle der Fig. 2 als auch der Fig. 3 zu einem Zweipol zusammengeschaltet sind, sind in vorteilhafter Weise nur zwei Leitungen 3, 15 zwischen dem Sensor 6 und der in dem Herzschrittmacher enthaltenen Ansteuerschaltung 7 erforderlich, deren eine außerdem dazu dient, dem zu stimulierenden Herzen 2 Stimulationsimpulse zuzuleiten bzw. der Detektoreinrichtung 5 die der elektrischen Aktivität des Herzens 2 entsprechenden Signale zuzuführen.

Obwohl die erfindungsgemäße Meßvorrichtung ausschließlich als Bestandteil eines Herzschrittmachers beschrieben wurde, sind beliebige andere Verwendungszwecke denkbar. In diesem Falle sind die Leuchtdiode 16, der Fototransistor 17 und der PTC-Widerstand 18 in das Ende eines Katheters integriert, der eine zweipolige Leitung enthält, die zur Verbindung der genannten Bauelemente mit der Ansteuerschaltung 7 dient.

Bezugszeichenliste

| | |
|---|---|
| 1 | Stimulationsimpuls-Generator |
| 2 | Herz |
| 3 | Leiter |
| 4 | Elektrode |
| 5 | Detektoreinrichtung |
| 6 | Sensor |
| 7 | Ansteuerschaltung |
| 8 | Steuerlogik |
| 9 | Quarz |
| 10 | Telemetrieschaltkreis |
| 11 | Sende/Empfangs-Spule |
| 12 | Gehäuse |
| 13, 14 | Steuerleitung |
| 15 | Leiter |
| 16 | Leuchtdiode |
| 17 | Fototransistor |
| 18 | PTC-Widerstand |
| 19 | Konstantstromquelle |
| 20 | Operationsverstärker |
| 21 | elektronischer Schalter |
| 22 | Konstantspannungsquelle |
| 23 | Shunt-Widerstand |
| 24 | Operationsverstärker |
| 25 | elektronischer Schalter |

**Patentansprüche**

1. Meßvorrichtung zur intrakardialen Erfassung eines der körperlichen Aktivität eines Lebewesens entsprechenden Meßsignales, welche eine Einrichtung zur Bildung eines dem Blutsauerstoffgehalt entsprechenden Signalanteiles mit einem Lichtsender (16) zur Abstrahlung von Licht in das Blut des Lebewesens und einem Lichtempfänger (17) zum Empfangen des am Blut reflektierten Lichtes, einem Temperatursensor (18) zur Bildung eines der Bluttemperatur entsprechenden Signalanteiles und eine Ansteuerschaltung (7) aufweist, welche einen konstanten Meßstrom oder eine konstante Meßspannung abgibt, wobei der Lichtsender (16), der Lichtempfänger (17) und der Temperatursensor (18) zu einem Zweipol verbunden sind, durch den der Meßstrom fließt oder über dem die Meßspannung abfällt, und wobei sich das Meßsignal aus dem Spannungsabfall über der Ansteuerschaltung (7) oder aus dem der Ansteuerschaltung (7) entnommenen Strom ergibt.

2. Meßvorrichtung nach Anspruch 1, **dadurch gekennzeichnet,** daß Mittel (8) zum Verstellen des Meßstromes oder der Meßspannung vorgesehen sind.

3. Meßvorrichtung nach Anspruch 1 oder 2, **dadurch gekennzeichnet,** daß die Einrichtung zur Bildung des dem Blutsauerstoffgehalts entsprechenden Signalanteiles ein Bauteil (16) mit einem dem des Temperatursensors (18) entgegengerichteten Temperaturkoeffizienten aufweist und der Meßstrom oder die Meßspannung auf einen Wert einstellbar ist, bei dem das Meßsignal bluttemperaturunabhängig ist.

4. Meßvorrichtung nach einem der Ansprüche 1 bis 3, **dadurch gekennzeichnet,** daß der Meßstrom oder die Meßspannung auf einen Wert einstellbar ist, bei dem die optische Funktion der Einrichtung zur Bildung des dem Blutsauerstoffgehalt entsprechenden Signalanteiles so weit reduziert ist, daß das Meßsignal blutsauerstoffgehaltunabhängig ist.

5. Meßvorrichtung nach einem der Ansprüche 1 bis 4, **dadurch gekennzeichnet,** daß als Lichtsender und/oder Lichtempfänger Fotohalbleiter vorgesehen sind.

6. Meßvorrichtung nach einem der Ansprüche 1 bis 5, **dadurch gekennzeichnet,** daß als Temperatursensor (18) ein Bauteil mit einem temperaturabhängigen Widerstandswert vorgesehen ist.

7. Meßvorrichtung nach Anspruch 5 oder 6, **dadurch gekennzeichnet,** daß als Lichtsender eine Leuchtdiode (16) und als Temperatursensor ein zu dieser in Serie geschalteter PTC-Widerstand (18) vorgesehen ist, daß als Lichtempfänger ein Fototransistor (17) vorgesehen ist, der dem PTC-Widerstand (18) parallelgeschaltet ist, und daß die Ansteuerschaltung (7) einen konstanten Meßstrom liefert.

8. Meßvorrichtung nach einem der Ansprüche 1 bis 7, **dadurch gekennzeichnet,** daß die Ansteuerschaltung (7) den Meßstrom bzw. die Meßspannung pulsförmig abgibt.

9. Herzschrittmacher, **dadurch gekennzeichnet,** daß der Herzschrittmacher eine Meßvorrichtung nach einem der Ansprüche 1 bis 8 enthält und die Stimulationsfrequenz des Herzschrittmachers in Abhängigkeit von dem Meßsignal der Meßvorrichtung gesteuert wird.

10. Herzschrittmacher nach Anspruch 9, **dadurch gekennzeichnet,** daß Mittel (8) vorgesehen sind, welche den Meßstrom oder die Meßspannung in Abhängigkeit von der jeweils vorlie-genden Stimulationsfrequenz steuern.

FIG 1

FIG 2

FIG 3

EP 90 11 8714

**Europäisches Patentamt**

# EUROPÄISCHER RECHERCHENBERICHT

Nummer der Anmeldung

**EP 90 11 8714**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP-A-0 249 822  (SIEMENS A.G.)<br>* Spalte 1, Zeilen 19 - 35; Figuren * * Spalte 2, Zeile 17 - Spalte 3, Zeile 54 *<br>– – – | 1,3,5,6,8,<br>9 | A 61 B 5/00<br>A 61 N 1/36 |
| A | EP-A-0 247 296  (SIEMENS A.G.)<br>* Spalte 5, Zeile 49 - Spalte 6, Zeile 46 * * Figuren 4, 5 *<br>– – – – – | 1,5,9 | |

|  |  |  | RECHERCHIERTE SACHGEBIETE (Int. Cl.5) |
|---|---|---|---|
| | | | A 61 B<br>A 61 N |

**Der vorliegende Recherchenbericht wurde für alle Patentansprüche erstellt**

| Recherchenort | Abschlußdatum der Recherche | Prüfer |
|---|---|---|
| Den Haag | 04 Juni 91 | RIEB K.D. |